# FASCICULE DE BREVET EUROPEEN

(11) **EP 2 665 516 B1**
(45) Date de publication et mention de la délivrance du brevet: **02.09.2015**
(21) Numéro de dépôt: 12705348.6
(22) Date de dépôt: 20.01.2012
(51) Int. Cl.: A61N 2/02, A61N 1/40

(54) **DISPOSITIF POUR L'ÉMISSION D'UN CHAMP MAGNÉTIQUE**
VORRICHTUNG ZUM AUSSENDEN EINES MAGNETFELDES
DEVICE FOR EMITTING A MAGNETIC FIELD

(30) Priorité: 21.01.2011 FR 1150494; 21.01.2011 FR 1150496
(43) Date de publication de la demande: 27.11.2013
(73) Titulaire: Cosmosoft, 92300 Levallois-Perret (FR)
(72) Inventeur: GREFF, Daniel, F-78490 Mere (FR)
(74) Mandataire: Chantraine, Sylvie Hélène
(86) Numéro de dépôt international: PCT/FR2012/050127
(87) Numéro de publication internationale: WO 2012/098338

(56) Documents cités:
- US-A- 4 266 532
- US-A- 4 850 959
- US-A- 5 100 373
- US-A- 5 620 463
- US-A1- 2003 045 770
- US-A1- 2004 193 003
- US-A1- 2005 228 210
- US-A1- 2006 235 491
- US-A1- 2008 139 871
- US-B1- 6 261 221

## Description

### Arrière-plan de l'invention

La présente invention se rapporte au domaine général de l'utilisation des ondes électromagnétiques pour agir sur le métabolisme du corps humain en vue d'induire des changements physiologiques, en particulier pour favoriser le sommeil, augmenter la fertilité, et traiter les surcharges adipeuses.

Un champ électromagnétique est la composition de deux champs vectoriels que l'on peut mesurer indépendamment : un champ électrique et un champ magnétique. La composante du champ électrique, présente dans tout champ électromagnétique, devient significative à partir d'une fréquence de 1KHz, alors qu'à une fréquence de 50Hz par exemple, le champ magnétique prédomine. On utilisera dans la présente demande le terme de champ magnétique pour désigner un champ électromagnétique dont la composante magnétique est prépondérante.

L'application d'un champ électromagnétique de basse fréquence ou de haute fréquence pour réduire la masse adipeuse a été suggérée à maintes reprises dans l'art antérieur. Cependant, aucune méthode efficace contre le traitement de l'obésité n'a été trouvée jusqu'à présent.

La réduction de la masse graisseuse par application d'un champ magnétique a déjà été proposée. On pourra notamment se référer aux documents FR 2,855,415, FR 2,906,727 et EP 2,068,810.

Ces documents décrivent des dispositifs de traitement des surcharges adipeuses composés de sangles isolantes appliquées sur la partie du corps humain à traiter (abdomen, bras, cuisse ou mollet), ces sangles étant équipées d'un conducteur replié en brins successifs et relié à un générateur d'ondes électromagnétiques. Dans le document EP 2,068,810, le dispositif comprend en outre un appareil de pressothérapie superposé aux sangles.

Bien que présentant des résultats certains sur la réduction de la masse graisseuse corporelle, l'efficacité de ces dispositifs reste limitée. En effet, ces dispositifs ne stimulent la lipolyse qu'au niveau des tissus superficiels.

D'autres méthodes de traitement appliquées au corps humain qui mettent en oeuvre des champs électromagnétiques de basse fréquence ont été décrites.

Dans le document US 2010/0130945 notamment, on a proposé de prévenir les infarctus du myocarde en appliquant un champ magnétique dont on fait varier l'intensité en fonction de la zone du corps sur laquelle on l'applique. Le champ magnétique agirait sur les cellules musculaires des artères ou sur les myocytes du myocarde pour relâcher les muscles et améliorer l'irrigation sanguine.

Le document US 2007/0255085 enseigne la stimulation des tissus nerveux d'un patient en combinant des ultrasons à deux champs magnétiques, essentiellement dans le but d'atténuer la douleur. Dans le document US 5 100 373, il été proposé de traiter l'ostéoporose en appliquant un champ magnétique de fréquence égale à 16 Hz et d'amplitude égale à 20,9 ou 12,7 microT.

Enfin, le document WO 2008/127011 décrit l'irradiation complète du corps par un champ magnétique d'intensité comprise entre 3 et 30 microT, pour agir sur la bipolarisation des membranes cellulaires et stimuler notamment les neurones, les cellules musculaires et les parois des vaisseaux sanguins. Ce document suggère de mettre en oeuvre cette méthode pour soigner un grand nombre de maladies, dont l'obésité. La fréquence du champ magnétique appliqué est choisie comme étant égale à la fréquence de résonance des molécules d'eau (12,5Hz, 16,3 Hz, 23,4 Hz, 39,6 Hz, 87 Hz ou 250 Hz) ou à l'une des fréquences des ondes cérébrales conditionnant la croissance, la concentration ou la mémorisation par exemple.

L'application d'un champ électromagnétique de haute fréquence pour réduire la masse adipeuse a également été proposée.

Il a été décrit notamment dans la demande de brevet US 2009/0125013 une méthode de réduction des tissus adipeux par application d'ondes électromagnétiques de très haute fréquence, entre 1 et 50 MHz. Selon cette méthode, on adapte la fréquence et la polarité d'un champ électrique à l'impédance des cellules adipeuses pour détruire les globules lipidiques qui se trouvent à l'intérieur des cellules ou pour détruire la membrane cellulaire des adipocytes. La forme du champ électrique est générée par l'association de deux antennes émettant respectivement dans le plan parallèle à la peau et dans le plan perpendiculaire de manière à pouvoir adapter la polarité du champ électrique à la forme de la zone adipeuse du corps à traiter.

Des méthodes de traitement de diverses maladies par thermothérapie ont également été proposées dans la demande de brevet US 2005/0090732. Selon cette méthode, la chaleur générée par un champ électromagnétique de fréquence comprise entre 0,1 et 900 MHz permettrait en théorie de cibler les cellules malades en élevant leur température entre 40 et 46°C, afin de les détruire. Toute une liste de maladies parmi lesquelles le cancer, le sida, la resténose, la tuberculose et l'obésité est donnée. Cette méthode n'a cependant pas fait ses preuves dans le traitement de l'obésité.

Un dispositif comprenant une série de bobines plates coplanaires qui se chevauchent et qui sont alimentées individuellement par un courant pulsé, a déjà été proposé dans la demande US 2003/0158583 pour stimuler les nerfs ou contracter les muscles et traiter divers désordres biologiques par un champ électromagnétique de haute fréquence dont la composante électrique est prépondérante. Cependant, ce dispositif est conçu pour générer un champ électrique diffus toujours dirigé vers le centre du corps, si bien qu'il ne permet pas de cibler une zone du corps particulière. En outre, ce dispositif présente l'inconvénient de manquer de sécurité à l'usage, car les bobines peuvent facilement glisser les unes par rapport aux autres, et les tensions de courant utilisées pour le faire fonctionner sont très élevées.

Le document WO 1996/16692 décrit également un dispositif sous forme de bobine pour générer un champ électromagnétique de très haute fréquence, typiquement entre 20 et 50 kHz, et de très haute intensité de l'ordre de 2 Tesla, pour contracter les muscles abdominaux ou stimuler les nerfs. Ce dispositif présente des risques pour la santé car il expose les organes du corps tels que le coeur, les poumons ou le cerveau à des ondes électromagnétiques trop intenses.

### Objet et résumé de l'invention

La présente invention a donc pour but principal de pallier de tels inconvénients en proposant un dispositif particulièrement efficace dans son action sur le corps, notamment pour la réduction des surcharges adipeuses, et inoffensif pour la santé de la personne exposée.

Ce but est atteint grâce à un dispositif pour exposer au moins une partie du corps d'une personne à un champ magnétique comprenant, conformément à l'invention, une ceinture isolante destinée à être appliquée en contact d'une partie du corps d'une personne, au moins une antenne plate dipolaire de forme sensiblement rectangulaire émettant un champ magnétique suivant un vecteur perpendiculaire à sa surface, l'antenne étant disposée longitudinalement sur la ceinture dans le sens de la longueur de celle-ci, et une source d'alimentation en courant alternatif ou pulsé de l'antenne.

Le ou les antennes du dispositif selon l'invention émettent un champ magnétique dirigé localement vers certaines zones du corps à traiter, à savoir en particulier la ceinture abdominale, les bras, les cuisses, les mollets et le dos. L'application de ce champ magnétique permet de simuler une activité sportive, notamment par son action sur les canaux calciques, et ce sans contraction musculaire ressentie par le patient. D'autre part, il a été découvert que ce dispositif permet non seulement de réduire la masse graisseuse viscérale ou intramusculaire, mais aussi de lutter contre l'obésité, de lutter contre le syndrome métabolique, et d'augmenter de façon très significative les chances de procréation médicalement assistée chez des personnes stériles.

Par ailleurs, le dispositif selon l'invention présente comme particularité que le champ magnétique est émis par des antennes dipolaires plates et de forme rectangulaire qui sont disposées longitudinalement dans le sens de la longueur de la ceinture. Aussi, lorsque le dispositif est par exemple appliqué à la ceinture abdominale, la composante principale de ce champ magnétique n'est pas coaxiale à la colonne vertébrale de la personne et est très faible lorsque le champ atteint les zones critiques du corps (coeur, poumon, cerveau). De la sorte, l'utilisation du dispositif selon l'invention ne présente que peu de risques pour la santé de la personne.

Le courant alternatif ou pulsé d'alimentation de l'antenne peut présenter une fréquence comprise entre 10 et 100 Hz, de préférence comprise entre 40 et 60 Hz. Elle peut être égale à 16 Hz, 32 Hz, 48 Hz, 64 Hz, 80 Hz ou 96 Hz, de préférence de l'ordre de 50 Hz. Par courant alternatif, on entend que la valeur et le sens du courant instantané changent. Par courant pulsé, on entend que seule la valeur du courant instantané change, son sens (négatif ou positif) restant toujours le même. Bien que possible, l'utilisation d'un courant pulsé présente néanmoins des résultats moins performants qu'avec le recours à un courant d'alimentation alternatif.

De plus, lorsque le courant d'alimentation de l'antenne est un courant alternatif, celui-ci présente de préférence une tension de l'ordre de 5 à 25 V, par exemple de 10 ou 20 V environ, et une intensité comprise entre 0,3 et 0,8 A, par exemple de l'ordre de 0,5 V. En effet, il a été constaté qu'un courant d'alimentation présentant de telles caractéristiques permet d'obtenir les meilleurs résultats en termes de densité de champ magnétique émis ainsi que de densité d'énergie évitant de placer le patient dans une situation de risques électriques ou thermiques.

La tension du courant d'alimentation du dispositif est de préférence comprise entre 5 et 25 V, de préférence entre 5 et 15 V. Elle peut aussi être égale à 20 V dans certains modes de réalisation. L'intensité du courant d'alimentation du dispositif est de préférence comprise entre 0,3 et 0,8 V, de préférence encore entre 0,5 et 0,8 V, plus préférentiellement égale à 0,5 V.

De façon avantageuse, l'antenne est dimension née pour émettre un champ magnétique dont l'intensité est au maximum de 200 microTesla (200 µT) environ. Il a en effet été constaté que l'application d'un champ magnétique d'intensité supérieure à 200 µT (soit 2 Gauss) peut présenter, à hautes doses, des effets secondaires nuisibles sur les cellules du cerveau et des os de la personne. La limitation à un tel seuil d'intensité de 200 µT permet donc d'éviter de tels risques.

Selon un mode de réalisation, la ceinture est une ceinture abdominale ayant deux rangées parallèles d'antennes plates dipolaires, chaque rangée étant composée d'une antenne centrale disposée entre deux antennes latérales, les antennes d'une même rangée étant alimentées en série.

Dans ce cas, les antennes d'une même rangée sont de préférence alimentées en courant alternatif de sorte à ce que l'antenne centrale émette un champ magnétique suivant un vecteur opposé en direction au vecteur suivant lequel est émis le champ magnétique émis par les antennes latérales.

Selon un autre mode de réalisation, la ceinture est une ceinture destinée à être portée autour du bras ou du mollet d'une personne, ladite ceinture ayant une unique antenne plate dipolaire.

Selon encore un autre mode de réalisation, la ceinture est une ceinture destinée à être portée autour de la cuisse d'une personne, ladite ceinture ayant deux rangées parallèles d'antennes plates dipolaires, chaque rangée étant composée de deux antennes latérales alimentées en série.

Selon un autre mode de réalisation, la ceinture est sous la forme d'un tapis sur lequel la personne s'allonge. Le tapis peut être posé sur le matelas d'un lit. Il peut également servir de tapis de relaxation ou de gymnastique. Lorsque la ceinture prend la forme d'un tapis, on utilise avantageusement au moins deux antennes, de préférence au moins quatre, de préférence encore au moins six, disposées parallèlement dans le sens de leur longueur. Le tapis sera de préférence placé de telle sorte que les antennes soient situées à proximité immédiate du dos, de préférence encore du bas du dos vers le milieu du dos.

Le dispositif de l'invention peut comprendre en outre un boîtier de contrôle de la source d'alimentation en courant alternatif de l'antenne et au moins un capteur de mesure du champ magnétique disposé sur la ceinture et relié audit boîtier de contrôle. La présence d'un ou plusieurs capteurs de mesure du champ magnétique permet de déterminer avec précision le seuil d'induction magnétique appliquée à la personne. En fonction de ces mesures, un réglage peut donc être réalisé à l'aide du boîtier de contrôle.

### Brève description des dessins

D'autres caractéristiques et avantages de la présente invention ressortiront de la description faite ci-dessous, en référence aux dessins annexés qui en illustrent des exemples de réalisation dépourvus de tout caractère limitatif. Sur les figures :
- la figure 1 est une vue d'une personne muni de dispositifs selon différents modes de réalisation de l'invention ;
- la figure 2 est une vue du dispositif de la figure 1 sous forme de ceinture abdominale ;
- la figure 3 est une vue en coupe longitudinale du champ magnétique émis par l'une des antennes du dispositif de la figure 2 ;
- la figure 4 est une vue en coupe longitudinale du champ magnétique émis par l'ensemble des antennes du dispositif de la figure 2 ;
- la figure 5 est une vue du dispositif de la figure 1 appliqué aux bras de la personne ; et
- la figure 6 est une vue du dispositif de la figure 1 appliqué aux cuisses et/ou aux mollets de la personne.

### Description détaillée de modes de réalisation

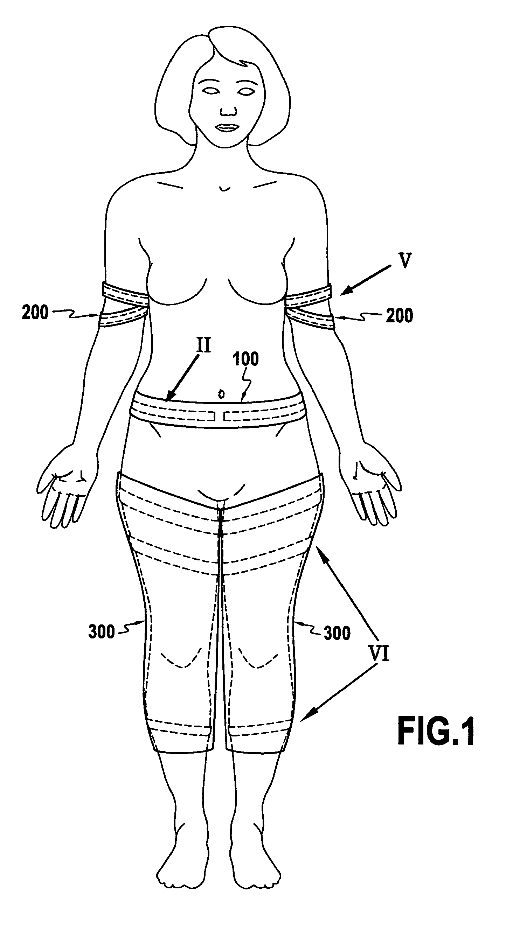

Le dispositif selon l'invention peut être appliqué à différentes parties du corps d'une personne. La figure 1 illustre ainsi plusieurs dispositifs, à savoir : un dispositif 100 disposé autour de la ceinture abdominale d'une personne, des dispositifs 200 disposés autour des bras de la personne, et des dispositifs 300 disposés autour de la cuisse et du mollet de chaque jambe de la personne.

Le dispositif peut également être placé sur le dos d'une personne, de préférence entre le bas et le milieu du dos.

Ces dispositifs 100 à 300 ont pour but d'émettre vers ces parties du corps de la personne des champs magnétiques dont les caractéristiques (notamment de direction et d'intensité) visent à réduire la masse graisseuse présente au niveau de ces parties. Cette réduction des surcharges adipeuses est rendue possible grâce à l'effet du champ magnétique généré par le dispositif selon l'invention qui simule une pratique sportive.

Quelle que soit la partie du corps sur laquelle il est appliqué, le dispositif selon l'invention comprend une ceinture isolante, au moins une antenne plate dipolaire de forme sensiblement rectangulaire émettant un champ magnétique suivant un vecteur perpendiculaire à sa surface, l'antenne étant disposée longitudinalement sur la ceinture dans le sens de la longueur de celle-ci, et une source d'alimentation en courant alternatif de l'antenne.

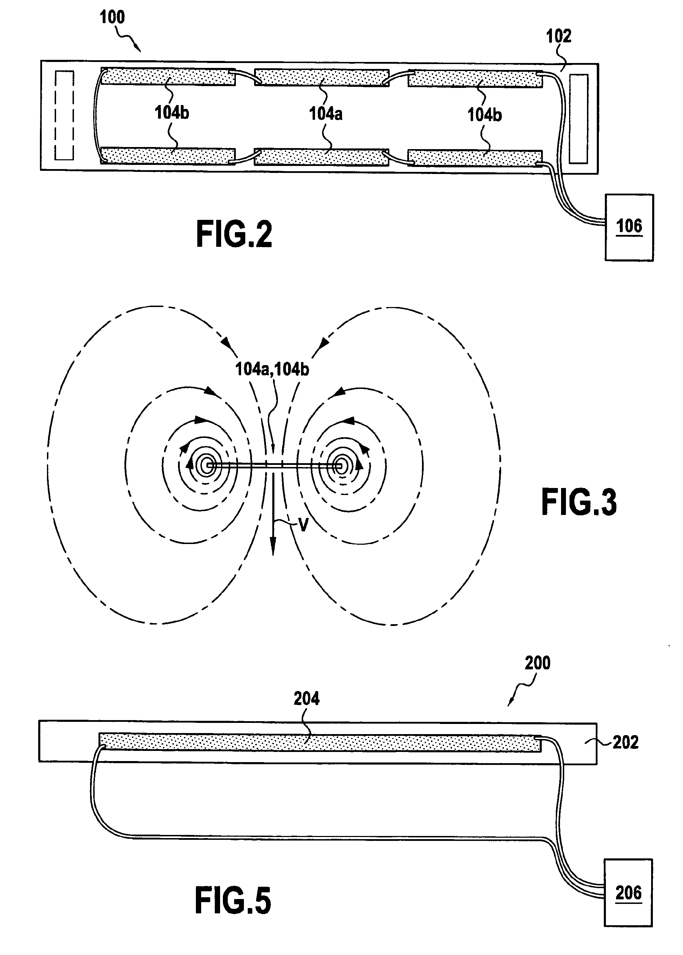

Ainsi, comme représenté sur la figure 2, le dispositif 100 destiné à être appliqué autour de la ceinture abdominale comprend une ceinture 102 réalisée dans un matériau isolant, par exemple en polyester enduit de PVC, et pouvant se fermer à la manière d'une ceinture vestimentaire.

La ceinture 102 comprend deux rangées parallèles d'antennes plates dipolaires, chaque rangée d'antennes étant composée d'une antenne centrale 104a disposée entre deux antennes latérales 104b. Les antennes sont plus précisément de forme sensiblement rectangulaire et disposées dans le sens de la longueur de la ceinture 102 (elles s'étendent longitudinalement dans le sens de la longueur de la ceinture comme représenté sur la figure 2). Par ailleurs, elles peuvent par exemple être disposées entre deux feuilles de matériau isolant constituant la ceinture. Par le terme « ceinture », on entend également couvrir la forme d'un tapis ainsi que d'une couverture ou duvet, sur ou dans lequel le patient peut être positionné. La couverture peut envelopper les jambes et comprendre, selon un mode de réalisation, une séparation des deux jambes qui sont donc couvertes séparément.

Chaque antenne 104a, 104b est une antenne rectangulaire plate ayant un pôle électromagnétique à chacune de ses extrémités longitudinales.

Elle est avantageusement réalisée dans un matériau souple et fin, par exemple dans une feuille d'un matériau polymère d'épaisseur de l'ordre de 10-50 microns, de préférence de 30-40 microns. L'épaisseur du matériau est choisie de telle sorte que la dissipation d'énergie qui se produit au cours du fonctionnement de l'antenne ne provoque pas une élévation trop importante de sa température, et ne gêne pas la personne.

Dans un mode de réalisation, l'antenne est un rectangle de polyimide sur lequel est gravé une ligne de métal conducteur, typiquement du cuivre, et présente comme caractéristique principale d'émettre, lorsque alimentée en courant alternatif, un champ magnétique suivant un vecteur perpendiculaire à sa surface.

La ligne de conducteur est dessinée le long du périmètre du rectangle et s'enroule sur plusieurs tours non jointifs vers l'intérieur, si bien que les spires ne sont pas en contact les unes avec les autres. Pour limiter la densité de courant dans le dispositif, le conducteur électrique comprend au maximum une dizaine de tours afin d'assurer à la fois i) la sécurité de l'usager (limiter une élévation de température trop grande pouvant conduire à des brûlures) et ii) une intensité du champ magnétique suffisante. Un procédé de gravure au cuivre sur polyimide est connu de l'homme du métier et ne sera donc pas décrit ici en détails, l'utilisation de procédés de gravures de circuits imprimés souples en polyimide étant répandue dans l'industrie.

Ces antennes permettent de générer un champ dont la forme est assimilable à un cornet dont la section est rectangulaire.

La longueur de l'antenne est de préférence comprise entre 10 et 60 cm, de préférence encore entre 20 et 50 cm, et de préférence encore entre 30 et 40 cm. Une dimension de l'ordre de 35cm de longueur par exemple permet de disposer d'un flux relativement homogène quant à sa densité de croissance/décroissance latéralement.

La largeur de l'antenne est de préférence comprise entre 1,5 et 10 cm, de préférence entre 2 et 6 cm. Une largeur de l'ordre de 3cm permet notamment une ouverture conditionnant la décroissance rapide de la puissance du champ magnétique en fonction de la profondeur dans le corps. Au-delà de 3cm de profondeur, seuls 10% à 20% de la puissance magnétique subsistent.

Le ratio entre la longueur et la largeur de l'antenne est de préférence compris 1,1 :1 et 20 :1. Il est de préférence compris entre 5 :1 et 15 :1, typiquement de l'ordre de 10 :1. Cette caractéristique de ratio largeur par longueur permet de résoudre les problématiques de sécurité intrinsèque du dispositif, aussi bien en terme électrique si le dispositif était abimé, qu'en termes de champ magnétique et de conformité des puissances magnétiques dans des sections du corps limitées, sur des profondeurs suffisantes mais limitées.

La figure 3 représente, en coupe longitudinale, la forme des lignes du champ magnétique émis par chacune de ces antennes 104a, 104b lorsqu'elle est alimentée en courant alternatif. Sur cette figure, on constate bien que chaque extrémité longitudinale de l'antenne constitue un pôle autour duquel se forment des lignes de champ et que la direction générale du champ magnétique est donnée par un vecteur V perpendiculaire à la surface de l'antenne.

Les antennes 104a, 104b du dispositif 100 pour la ceinture abdominale sont alimentées en courant alternatif ou pulsé au moyen d'une source d'alimentation 106 externe à la ceinture.

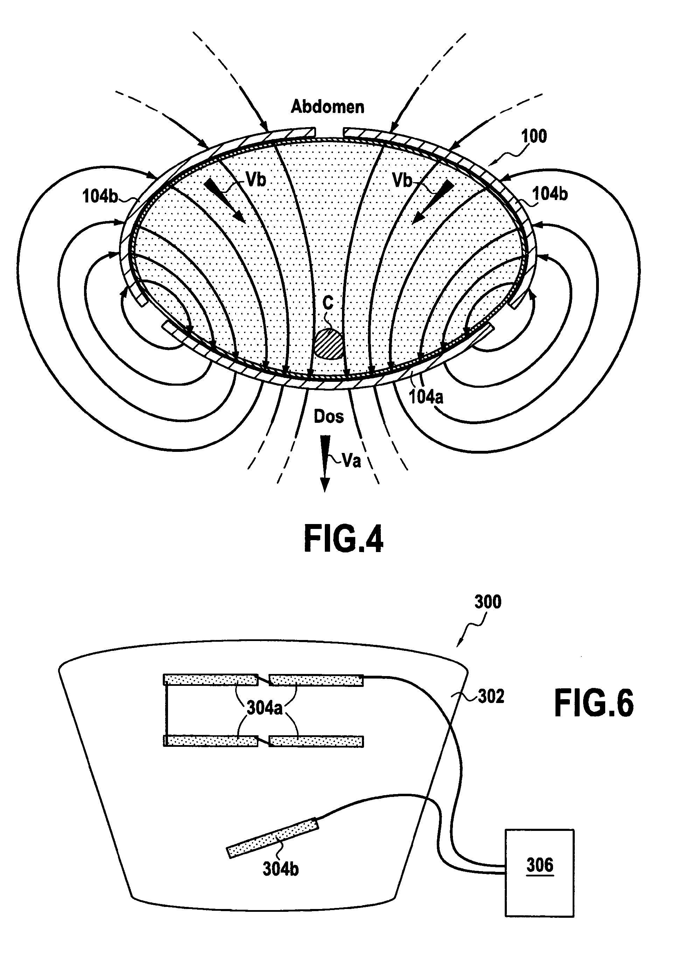

Par ailleurs, les antennes sont reliées électriquement entre elles de sorte que les antennes d'une même rangée soient alimentées en série. De plus, il est prévu que l'alimentation soit réalisée de sorte que les antennes latérales 104b d'une même rangée émettent un champ magnétique suivant un vecteur Vb opposé en direction au vecteur Va suivant lequel est émis le champ magnétique émis par l'antenne centrale 104a (voir figure 4).

De la sorte, lorsque la ceinture est fermée autour de la ceinture abdominale d'une personne, les lignes de champ de chaque rangée d'antennes ont la forme représentée sur la figure 4.

Sur cette figure, est représentée (en coupe) la ceinture abdominale du corps d'une personne portant le dispositif 100 selon l'invention, avec l'abdomen et le dos (contre lequel se situent les antennes centrales 104a du dispositif). On constate que les lignes de champ magnétique provenant des antennes d'une même rangée présentent une intensité maximale en surface de peau pour décroître en profondeur du corps.

Le champ magnétique ainsi généré par le dispositif permet donc un traitement optimum des zones musculaires situées à proximité des hanches et au niveau des abdominaux (l'intensité du champ étant concentrée dans ces zones). Plus en profondeur, l'intensité du champ magnétique étant réduite, les organes de la personne, et en particulier la colonne vertébrale C, sont épargnés par l'émission du champ magnétique.

De plus, les antennes du dispositif émettent un champ magnétique selon un vecteur perpendiculaire à leur surface, le champ magnétique rayonné en dessous et au dessus de la ceinture abdominale étant extrêmement faible. Les organes critiques de la personne tels que le coeur et les poumons sont donc également épargnés.

Pour obtenir de tels résultats, le courant alternatif ou pulsé d'alimentation des antennes 104a, 104b du dispositif présente une fréquence comprise entre 10 et 100 Hz, de préférence comprise entre 40 et 60 Hz. Elle peut être égale à 16 Hz, 32 Hz, 48 Hz, 64 Hz, 80 Hz ou 96 Hz, de préférence de l'ordre de 50 Hz. De plus, lorsqu'il est alternatif, le courant d'alimentation présente avantageusement une tension comprise entre 5 et 25 V, de préférence entre 5 et 15 V. Elle peut aussi être égale à 20 V dans certains modes de réalisation. L'intensité du courant d'alimentation du dispositif est de préférence comprise entre 0,3 et 0,8 V, de préférence encore entre 0,5 et 0,8 V, plus préférentiellement égale à 0,5 V.

De même, chaque antenne du dispositif est dimensionnée pour émettre, lorsqu'elle est alimentée par un courant présentant de telles caractéristiques, un champ magnétique dont l'intensité est au maximum de 200 µT environ. Une fois le dispositif appliqué au contact de la personne, le champ magnétique de 200 µT au maximum en surface de peau et ne dépassera pas 1 µT au centre du corps. Il a en effet été constaté que l'application d'un champ magnétique d'intensité supérieure à 200 µT pouvait présenter, à hautes doses, des effets secondaires nuisibles sur les cellules du cerveau et des os de la personne. La limitation à un tel seuil d'intensité de 200 µT permet donc d'éviter de tels risques.

En liaison avec la figure 5, on décrira maintenant le dispositif 200 destiné à être disposé autour d'un bras de la personne.

Ce dispositif 200 est identique à celui décrit en liaison avec la ceinture abdominale. En particulier, il comprend une ceinture 202 en matériau isolant, une seule antenne plate dipolaire 204 de forme rectangulaire (identique à celle dont les lignes de champ sont représentées sur la figure 3), et une source 206 d'alimentation de l'antenne en courant alternatif.

L'antenne 204 s'étend longitudinalement dans le sens de la longueur de la ceinture 202 comme représenté sur la figure 5. Les caractéristiques de l'antenne et de la source d'alimentation de celle-ci sont identiques à celles décrites en liaison avec le dispositif pour application sur la ceinture abdominale.

Ici, la ceinture 202 est enroulée en hélicoïde autour du bras de la personne. Cette disposition permet à l'antenne 204 d'émettre un champ magnétique dont les lignes de champ « traversent » le bras en étant annulées par celles de l'arrière du segment d'antenne opposé.

De la sorte, l'intensité du champ magnétique émis par l'antenne peut être régulée en fonction du diamètre du bras de la personne. Si le patient a un bras de petit diamètre, l'influence du champ magnétique provenant du côté de l'antenne qui est opposé à celui enroulé est élevé et en direction opposée à celui-ci. Il permet ainsi d'annuler en partie le champ émis vers les tissus de la personne. Au contraire, si le patient a un bras de gros diamètre, l'antenne du dispositif est disposée de façon quasiment verticale par rapport à ce diamètre. L'antenne s'approche alors d'un simple élément radiant vertical dont la distance entre les extrémités devient peu significative grâce à la forme hélicoïdale de son enroulement.

En liaison avec la figure 6, on décrira maintenant le dispositif 300 destiné à être disposé autour de la cuisse et du mollet d'une jambe de la personne.

Ce dispositif 300 est identique aux précédents. En particulier, il comprend une ceinture 302 en matériau isolant, cinq antennes plates dipolaires 304a, 304b décrites ci-dessous, et une source 306 d'alimentation en courant alternatif de ces antennes.

Les antennes sont identiques à celles dont les lignes de champ sont représentées sur la figure 3. Elles se composent, d'une part de deux rangées parallèles d'antennes plates dipolaires, chaque rangée étant composée de deux antennes latérales 304a alimentées en série, et d'autre part d'une antenne seule 304b.

Les antennes 304a disposées en rangées s'étendent longitudinalement dans le sens de la longueur de la ceinture 302 et sont destinées à venir entourer la cuisse de la personne de la même façon que les antennes du dispositif appliqué à la ceinture abdominale. Quant à l'antenne seule 304b, elle s'étend également longitudinalement dans le sens de la longueur de la ceinture et est destinée à être disposée autour du mollet correspondant de la personne de la même façon que l'antenne du dispositif appliqué au bras.

Le dispositif 300 a été décrit ici dans une application à la fois à la cuisse et au mollet d'une jambe d'une personne. Il est toutefois envisageable de réaliser un dispositif distinct pour chacune de ces parties, à savoir un dispositif destiné à être appliqué sur la cuisse et un dispositif distinct destiné à être appliqué sur le mollet.

On décrira maintenant certaines caractéristiques avantageuses communes à l'ensemble des dispositifs 100 à 300 précédemment décrits.

En particulier, ces dispositifs comprennent de préférence un boîtier de contrôle de la source d'alimentation en courant alternatif de l'antenne (non représenté). Ce boîtier de contrôle permet ainsi de contrôler l'intensité du champ magnétique émis pour le ou les antennes du dispositif.

Par ailleurs, toujours de façon avantageuse, ces dispositifs comprennent en outre au moins un capteur de mesure du champ magnétique (non représenté) disposé sur la ceinture et relié au boîtier de contrôle. La présence d'un ou plusieurs capteurs de mesure du champ magnétique permet de déterminer avec précision le seuil d'induction magnétique appliquée au patient, qui correspond à l'intensité optimale du courant électrique à choisir. En fonction de ces mesures, un réglage peut donc être réalisé à l'aide du boîtier de contrôle.

En particulier, les dispositifs peuvent comprendre un moyen de réglage automatique de l'intensité du champ magnétique émis pour le ou les antennes en fonction de la morphologie des usagers. Ceci est réalisé par exemple en compensant la perméabilité magnétique des personnes que les dispositifs peuvent ajuster. Une dichotomie sur l'intensité du champ magnétique émis par les antennes permet de trouver le point de fonctionnement idéal. La dichotomie converge lorsque le champ magnétique mesuré du côté opposé d'une antenne est égal à par exemple 0,01 Gauss (1µTesla).

L'invention porte également sur l'utilisation du dispositif dans des méthodes de traitement esthétique, des méthodes de relaxation ou des méthodes de simulation d'une activité sportive.
Le dispositif de l'invention est utilisé pour l'émission d'un champ magnétique pénétrant dans les tissus d'au moins une partie d'au moins un membre et/ou du tronc d'une personne.

Par « membre », on entend en particulier un ou les bras et une ou les jambes, et notamment le ou les mollets et le ou les cuisses, et en particulier la partie supérieure de la ou les cuisses.

Par « tronc » on entend en particulier la zone abdominale, la zone lombaire et le cou.

Le faible niveau de champ magnétique requis en surface de la peau, entre un dixième et quelques fois le champ magnétique terrestre (0,5 Gauss équivalent à 50 microT), permet la mise en oeuvre de ces techniques sans risques pour les cellules du corps humain, ni contraintes d'exploitation médicales.

Le champ magnétique appliqué aux tissus vivants permet, notamment par son action sur les canaux calciques des cellules, de simuler une activité sportive sans que le patient ne ressente de contraction musculaire.

Ainsi l'invention concerne une méthode de simulation de pratique sportive caractérisée en ce qu'elle comprend la mise en oeuvre d'un champ magnétique sur au moins une partie d'au moins un membre et/ou du tronc d'une personne, au moyen du dispositif décrit précédemment.

L'invention concerne également une méthode de relaxation musculaire ou d'amélioration de la qualité du sommeil, laquelle méthode comprend la mise en oeuvre d'un champ magnétique sur au moins une partie d'au moins un membre et/ou du tronc d'une personne au moyen du dispositif décrit précédemment.

Dans ces deux méthodes, le dispositif peut prendre la forme d'une ceinture, d'un bracelet, d'une couverture ou d'un tapis.

Le dispositif de l'invention induit très probablement une stimulation des canaux calciques au niveau musculaire, c'est-à-dire au niveau des fibres musculaires, sous l'influence du champ magnétique. Les méthodes de l'invention comprennent donc vraisemblablement une activation du cycle énergétique, notamment par hydrolyse d'ATP avec libération consécutive d'énergie et contraction des fibres musculaires, qui provoque la lipolyse des graisses sous-jacentes, pour reconstituer le stock d'ATP. La contraction des fibres musculaires n'est pas ressentie par la personne. De ce que comprend l'inventeur, le patient ne ressent pas ou quasiment pas les contractions musculaires qui sont stimulées par voie chimique par action sur les canaux calciques du fait de l'action du champ magnétique.

Les Ca²⁺-ATPases sont des enzymes membranaires localisées dans la membrane du réticulum sarcoplasmique des cellules musculaires où elles représentent 90% des protéines membranaires. Le réticulum sarcoplasmique stocke les ions Ca²⁺ et le flux rapide de ces ions du réticulum vers le sarcoplasme (cytoplasme des fibres musculaires) provoque la contraction musculaire. Il existe deux catégories principales de muscles:
- Les muscles striés ou squelettiques (40% du poids du corps, ils sont rattachés au squelette) dont la contraction est de nature électrique (par voie nerveuse, sous le contrôle du cerveau)
- Les muscles lisses (10% du poids du corps) dont la contraction, involontaire et insensible contrairement aux muscles squelettiques, est de nature chimique.
Cette contraction liée au couple Ca²⁺/ATP, est quatre fois plus rapide que la contraction des muscles striés. La contraction des muscles lisses consomme énormément d'énergie, elle est le mode de fonctionnement des principaux viscères tels que les intestins, les vaisseaux sanguins, l'utérus et les reins. La stimulation magnétique des ions Ca²⁺ qui provoque une augmentation de l'activité de ces ATPases entraine de facto une stimulation de la lipolyse des graisses stockées au niveau des muscles lisses et striés. Dans le cas des muscles squelettiques, le surplus d'ATP généré par cette stimulation magnétique, non accompagnée d'une contraction musculaire, sera éliminé par le corps, de la même manière qu'un sportif le fait en récupération après l'effort. Dans le cadre de la présente invention, le champ électromagnétique appliqué à la surface de la peau, pénètre dans le derme et l'espace corporel et fait entrer en résonnance les ions calcium. L'application du champ électromagnétique par le dispositif de l'invention ne provoque pas la destruction des adipocytes par rupture de leur membrane. Elle permet au contraire de stimuler la lipolyse, c'est-à-dire le mécanisme naturel de la consommation des graisses contenues dans les adipocytes essentiellement situés autour des viscères et des muscles.

L'étude réalisée dans le cadre de l'invention sur puces à ADN, a démontré de façon indéniable la stimulation génique des canaux calciques au niveau musculaire sous l'influence du champ magnétique généré pas le dispositif. Grâce au dispositif de l'invention, il n'est pas nécessaire d'imposer en surface de la peau un champ magnétique d'intensité élevée pour assurer en profondeur une intensité suffisante pour activer les cibles biologiques. Les dispositifs de l'art antérieur ne permettent pas d'agir à une telle profondeur sans mettre en cause la sécurité de la personne. En effet, le dispositif de la présente invention permet une action ciblée du champ magnétique qui rayonne essentiellement entre les deux plans qui sont perpendiculaires à la surface de l'antenne et qui passent par ses longueurs. Il n'y a pas de diffusion du champ magnétique sur une zone du corps qui ne doit pas être exposée à un champ magnétique, telle que le coeur, le cerveau ou les poumons.

Selon un mode de mise en oeuvre particulier, la méthode permet de remodeler la silhouette ou d'améliorer l'esthétique du corps d'une personne saine, i.e. qui n'est atteinte d'aucune maladie déclarée en rapport avec une surcharge de masse adipeuse. L'invention a ainsi comme objet une méthode non-thérapeutique dans un but esthétique pour la réduction de la masse graisseuse viscérale ou intramusculaire qui comprend la mise en oeuvre d'un champ magnétique sur au moins une partie d'au moins un membre et/ou du tronc d'une personne au moyen d'un dispositif décrit plus haut, en particulier d'une personne saine.

Dans cette méthode, le dispositif peut prendre la forme d'une ceinture, d'un bracelet, d'une couverture ou d'un tapis. Il est de préférence sous la forme d'un ceinture telle que décrite à la figure 2.

La présente invention concerne aussi une méthode de réduction de la masse graisseuse viscérale ou intramusculaire, c'est-à-dire au niveau des adipocytes intramusculaires, caractérisée en ce qu'elle comprend la mise en oeuvre d'un champ magnétique sur au moins une partie d'au moins un membre et/ou du tronc d'une personne. La réduction de la masse graisseuse en profondeur par la méthode de la présente invention permet une amélioration très efficace de l'esthétique de la personne, par exemple de sa silhouette.

L'invention concerne également une méthode mettant en oeuvre un champ magnétique sur au moins une partie d'au moins un membre et/ou du tronc d'une personne au moyen du dispositif décrit précédemment pour le traitement ou la prévention de troubles choisis parmi la stérilité, l'obésité et le syndrome métabolique.

Selon ces aspects de l'invention, le dispositif peut prendre la forme d'une ceinture, d'un bracelet, d'une couverture ou d'un tapis. Il est de préférence sous la forme d'un ceinture telle que décrite à la figure 2.

Le syndrome métabolique détecté chez une personne n'ayant déclaré aucun symptôme particulier correspond à un risque de désorganisation métabolique ou de maladie supérieur à un individu dit en bonne santé. On peut ainsi définir le syndrome métabolique comme un risque d'accident cardio-vasculaire multiplié par trois par rapport à un individu réellement en bonne santé. Le syndrome métabolique décrit un état qui est considéré comme préfigurant plusieurs maladies graves dont:
- le diabète de type 2 (DT 2) ;
- les troubles cardiovasculaires ;
- les accidents vasculaires cérébraux (AVC).

Le dispositif de l'invention permet également de prévenir ou de lutter contre l'obésité, en particulier l'obésité abdominale, et par voie de conséquence contre les maladies associées à l'obésité. Aussi, la présente invention concerne également une méthode pour le traitement de l'obésité abdominale, caractérisée en ce qu'elle comprend la mise en oeuvre d'un champ magnétique sur au moins d'au moins un membre et/ou du tronc d'une personne au moyen du dispositif décrit précédemment.

Les dispositifs de l'art antérieur ne permettent pas de stimuler la lipolyse en profondeur dans les tissus. Or il a été découvert de manière surprenante que le champ magnétique généré par le dispositif de l'invention, en particulier selon les variantes et perfectionnements de la présente invention, provoque la consommation de la graisse située en profondeur, notamment sous le derme, dans les viscères ou les muscles. Le champ magnétique généré par l'antenne à la surface de la peau pénètre dans le corps jusqu'à une profondeur donnée, typiquement de l'ordre de 2 à 10 cm, de préférence à plus de 5 cm, sur toute la surface de l'antenne. Son intensité décroît avec la profondeur.

Enfin il a été découvert de manière inattendue qu'un champ magnétique présentant l'intensité et la fréquence mentionnées plus haut possède également une activité pour stimuler et/ou améliorer l'ovogenèse et la spermatogenèse. Ainsi la présente invention couvre également une méthode pour provoquer ou augmenter l'ovogenèse et/ou la spermatogenèse chez une personne ou un animal, en particulier un mammifère, en ayant besoin. La méthode de l'invention permet de traiter la stérilité, d'augmenter la fertilité, et de prévenir un risque de stérilité, notamment lorsqu'elle découle d'une obésité ou d'un risque d'obésité élevé contre lequel il faut lutter. La méthode de l'invention peut être appliquée aux femmes et aux hommes.
Dans le cadre de cette méthode tout dispositif connu de l'homme du métier pour émettre un champ magnétique dont l'intensité et la fréquence sont conformes à celles décrites plus haut peuvent être utilisés. On choisira de préférence le dispositif selon l'invention pour les avantages supplémentaires qu'ils procurent.

La fertilité est dépendante de la présence dans le corps de gamètes (ovocytes ou spermatozoïdes) et la stérilité peut avoir différentes causes, dont les troubles de la production de gamètes. Chez la femme en particulier la stérilité peut découler de troubles de l'ovulation (les ovaires polykystiques ou l'endométriose qui perturbe l'ovulation ou la nidation de l'embryon), de l'obstruction ou de l'altération des trompes, ou d'une anomalie de la glaire cervicale. Par « traitement ou prévention de la stérilité » au sens de la présente invention, on entend le traitement ou la prévention des troubles qui viennent d'être décrits, et en particulier la stimulation ou l'augmentation de l'ovogenèse ou de la spermatogenèse, notamment dans le cas où celle-ci est inexistante ou lorsqu'elle est insuffisante. Dans un mode de mise en oeuvre de l'invention, le traitement de la stérilité entraîne l'augmentation du taux de grossesse chez des personnes suivant un protocole de procréation médicalement assistée, par différentes techniques, par exemple par fécondation in vitro conventionnelle (FIV), par fécondation in vitro avec microinjection (ICSI : Intra cytoplasmic sperm injection), par microinjection avec spermatozoïdes choisis (IMSI : Morphologically Selected Sperm Injection ou SICSI : Scored Intra Cytoplasmic Sperm Injection).

L'invention a ainsi pour objet une méthode appliquant un champ magnétique susceptible d'être généré par le dispositif décrit plus haut pour améliorer la fertilité, en particulier chez des personnes souffrant d'obésité, en particulier chez des femmes ou des hommes présentant un Indice de Masse Corporelle (IMC) supérieur ou égal à 30. Il a été démontré par une étude en milieu hospitalier que des patientes obèses - jugées stériles à la suite d'examens d'exploration fonctionnelle et de bilans biologiques (dosage FSH/LH, réserve ovarienne, qualité ovocytaire, spermogramme) - peuvent de façon tout à fait inattendue améliorer leur chance d'avoir un enfant par la voie de procréation médicalement assistée. Dans cette méthode, le dispositif peut prendre toute forme possible, de préférence celle d'une ceinture abdominale. La durée des séances d'exposition au champ magnétique est généralement comprise entre 15 et 75 minutes. Elle peut être selon le résultat recherché de 30 ou de 60 minutes.

Dans toutes les méthodes qui viennent d'être décrites on adapte les paramètres de dimension et de forme de l'antenne, d'intensité et de tension du courant électrique alimentant l'antenne, de durée des séances d'exposition au champ magnétique, du nombre de séances en fonction du résultat recherché.

Avantageusement, le champ magnétique lorsqu'il est généré par un courant alternatif possède une fréquence comprise entre 10 et 100 Hertz, par exemple égale à 16 Hz, 32 Hz, 48 Hz, 50 Hz, 64 Hz, 80 Hz ou 96 Hz, et une intensité comprise entre 1 et 400 microT (0,01 et 4 Gauss), et de préférence entre 10 et 200 microT (0,1 et 2 Gauss). On préfère limiter l'intensité du champ électromagnétique généré en surface de la peau à 200 microTesla (2 Gauss).

Selon un mode de réalisation avantageux, la méthode comprend un contrôle du dosage d'un champ magnétique appliqué sur le patient. Ceci constitue en particulier une amélioration significative des dispositifs connus et décrits dans les brevets cités précédemment.
Ce contrôle du dosage est en particulier effectué par régulation de l'intensité du champ magnétique. Ce contrôle du dosage est facilement effectué par une variation de l'intensité du courant électrique alimentant l'antenne et générant le champ magnétique, qui est typiquement comprise entre 0,3 et 0,8 A.

Selon un de ses aspects, l'invention concerne également une méthode de contrôle du dosage d'un champ magnétique appliqué sur une personne, notamment dans un but de soin esthétique ou thérapeutique, caractérisée en ce qu'elle comprend la disposition d'un capteur d'ondes magnétiques à proximité immédiate d'une antenne rayonnante, ledit capteur d'ondes magnétiques étant en communication avec un boîtier de contrôle du champ magnétique, ledit boîtier de contrôle communiquant avec une ou plusieurs antennes rayonnantes, et la mesure par le boîtier de contrôle de la perméabilité magnétique de la personne afin d'adapter le champ magnétique émis par le ou les antennes rayonnantes en fonction d'un profil d'individu, notamment en diminuant ou augmentant l'intensité du courant.

Ceci permet notamment un dosage en intensité et en durée du champ magnétique appliqué à la personne.

L'invention concerne également selon un autre aspect une méthode de mesure de la réponse des tissus d'une personne à une mise en contact avec un champ magnétique, notamment dans un but d'optimisation d'un soin esthétique ou thérapeutique par champ magnétique, caractérisée en ce que ladite méthode comprend la variation des fréquences du champ électromagnétique, de préférence entre 10Hz et 10kHz, au regard d'une ou plusieurs parties d'un ou plusieurs membres et/ou d'une ou plusieurs parties du tronc d'une personne, et la mesure du champ électromagnétique pour estimer la réponse des tissus de la personne. La composante de champ électrique, présente dans tout champ électromagnétique, devient significative à partir de 1KHz, alors qu'à 50Hz le champ électromagnétique est surtout caractérisé par un champ magnétique prédominent. Ce champ électrique permet alors d'effectuer une mesure fine de la perméabilité magnétique.

Cette méthode permet notamment l'estimation de la masse graisseuse et/ou de la masse musculaire afin notamment d'adapter l'intensité et la durée du champ magnétique appliquée à la personne.

Cette méthode peut être par exemple mise en oeuvre en faisant passer un courant alternatif dans l'ensemble des antennes, puis l'extinction successive ou simultanée d'une ou plusieurs antennes pour mesurer le champ électromagnétique émis par les antennes restantes. Ainsi, en mesurant le champ électromagnétique émis au travers des tissus d'une personne, par exemple en positionnant le capteur électromagnétique de manière diamétralement opposée à l'antenne émettrice du champ à mesurer, le champ électromagnétique capté modélise la perméabilité magnétique de la personne. Pour la partie abdominale, on peut facilement ne laisser émettre que l'antenne dorsale 104a et mesurer le champ qui est reçu dans la partie abdominale. La ceinture du dispositif peut donc comprendre un capteur électromagnétique disposé de manière diamétralement opposée à au moins une antenne lorsque que la ceinture est mise en position sur le patient. Le capteur électromagnétique peut être une des antennes opposées, mais utilisée en réception pour détecter le niveau de signal traversant le patient.
Il est alors possible de distinguer des rapports de permittivité diélectrique de plusieurs ordres de magnitude entre 10Hz et 10KHz, mesurables réellement entre 1KHz et 10KHz. On peut réaliser une courbe de variation de permittivité diélectrique de la graisse (mesurée sur la poitrine par exemple) en fonction de la fréquence et une autre courbe de variation de permittivité diélectrique du tissu musculaire en fonction de la fréquence. La présence d'une pente de courbe type graisse plutôt que muscle permet d'évaluer les ratios de tissus à traiter. Cette information est complétée par la connaissance des poids et taille de la personne.

La méthode de l'invention comprend selon un mode de réalisation particulier l'estimation de la réponse des tissus de la personne en faisant varier les fréquences du champ magnétique sur une ou plusieurs parties d'un ou plusieurs membres et/ou parties du tronc de la personne.

Par exemple la mesure est effectuée par l'intermédiaire d'un capteur magnétique, et par exemple un capteur à effet hall ou magnéto résistif, notamment pour déterminer le seuil de puissance maximum et/ou optimum du champ magnétique à appliquer à la personne.

Pour mettre en oeuvre la méthode de l'invention, il est préférable d'émettre un champ magnétique alternatif ciblé sur certaines zones du corps, et notamment situées dans les zones de forte densité de muscles et/ou viscères. L'action du champ magnétique consiste à interagir avec certains éléments intervenant dans les cycles de consommation des énergies stockées dans le corps (graisses). En particulier les cycles organiques liés au Calcium sont suractivés (ATP ases, Ca²⁺).

L'effet du champ magnétique a été mesuré en effectuant un dosage de certains constituants sanguins avant et après une cure de 12 séances. Une analyse par puce ADN a permis de valider la chaine biochimique activée par le dispositif. Ceci a permis également de tracer une courbe d'étalonnage de l'intensité du champ magnétique appliqué en fonction de la morphologie de la personne, par exemple de son tour de taille, du diamètre de ses bras ou de ses jambes.

### EXEMPLES

### Exemple 1 : Réalisation d'un profil d'expression

Le but de l'étude a été la réalisation d'un profil d'expression pour la caractérisation des effets transcriptionnels induits sur des cellules sanguines de personnes soumises à un champ magnétique alternatif basse fréquence.

L'étude des réseaux de gènes a été effectuée par un moteur de recherche analytique.

Les RNAs totaux extraits de cellules sont traités afin d'obtenir des molécules fluorescentes. Ces molécules sont ensuite déposées sur les Microarrays et réagissent avec les séquences d'ADN des différents gènes correspondantes, sur la base de leur complémentarité. Ensuite on mesure la fluorescence grâce à un scanner spécifique et on peut ainsi quantifier la quantité de RNA produite dans la cellule dans la mesure où l'intensité mesurée est proportionnelle à la quantité de RNA qui interagit avec les séquences d'ADN déposées. Ces méthodes sont connues de l'homme de l'art.

### Protocole de l'étude :

- 11 personnes en surpoids sont sélectionnées pour l'étude. Les volontaires, 4 hommes et 7 femmes, avaient un âge compris entre 38 et 64 ans et présentaient, pour dix d'entre eux, une surcharge pondérale avérée.
- un premier prélèvement sanguin a été effectué pour l'ensemble des 11 personnes (bilan biologique prescrit par un médecin endocrinologue) avant le début de l'étude.
- un second prélèvement sanguin a été réalisé après les 12 séances.

Parmi les personnes de l'étude, 4 personnes (2 hommes, 2 femmes) ont effectué en plus un prélèvement au début et à la fin de la 8ème séance.

Les séances ont consisté en le positionnement de la partie inférieure du corps de la personne (jusqu'au thorax) dans une couverture renfermant les antennes du dispositif de la présente invention. La couverture présentait une séparation des jambes. Il était prévu trois antennes pour la partie abdominale, deux antennes pour chaque cuisse, une antenne pour chaque mollet, et une antenne pour chaque bras. L'antenne pour les mollets et les bras était disposée de manière hélicoïdale autour du membre. La couverture était reliée au boîtier de contrôle générateur du champ magnétique.

Le courant électrique alimentant le dispositif était conforme aux valeurs suivantes : la fréquence utilisée était de 50 Hz, la tension de 20 V et l'intensité comprise entre 500 et 800 mA.

Chaque séance a duré environ 60 minutes.

Les résultats ont été obtenus à partir d'une sélection de gènes répondant aux critères suivants :
(1) Les activations (induction supérieure à x 1,50 par rapport au témoin d'expression qui est l'expression du gène avant l'étude) ont été observées au moins chez 3 personnes sur 4, lors d'une analyse limitée aux personnes ayant effectué les 4 prélèvements.
(2) Il a été constaté que l'activation de ces gènes pour ces personnes a été maintenue au cours des différentes séances.
(3) Les gènes retenus n'ont été conservés que si leur modulation était retrouvée au moins pour 6 sujets sur 11.
(4) De faire partie d'un réseau fonctionnel biologique dans lequel plusieurs gènes présentant une modulation significative se retrouvent associés.

Cette étude a été effectuée à partir de l'étude du profil d'expression sur puces Agilent Whole Genome Microarray, comportant 43 776 séquences génétiques (hors contrôles).
- Ces puces ont été hybridées à des sondes correspondant aux échantillons d'ARNs extraits à partir de sang total. La qualité et la quantité de l'ensemble des échantillons des ARNs ont été mesurées.
- Un bilan biologique de contrôle a été réalisé pour l'ensemble des patients.

### Analyse des résultats :

Les données ont été soumises à une normalisation intra-puces et inter-puces (méthode quantile) au moyen d'un logiciel spécifique.

Les rapports des intensités : après les séances versus avant la première séance ont été calculés pour chaque personne afin de donner l'induction ou l'inhibition (modulation) de chaque gène étudier pour chaque personne.

Il a été effectué une sélection de gènes dont le rapport d'expression témoigne d'une modulation au moins chez trois des quatre personnes ayant bénéficié d'une analyse avant et après la 8ème séance.

Seuls les gènes qui présentaient une modulation aux conditions : avant/après la 8ème séance et après la 12ème séance ont été considérés.

Cette analyse a conduit à sélectionner 112 gènes qui ont été soumis à une étude de corrélation.

Dans un deuxième temps, les données d'intensités obtenues après normalisation ont été filtrées sur la base d'une intensité supérieure au bruit de fond pour l'ensemble des échantillons, et d'un rapport d'intensité supérieure à 1,5.

Cette sélection a identifié 89 gènes.

La pertinence des résultats obtenus a conduit à poursuivre l'analyse sur les 7 autres personnes. Parmi ces 7 personnes, 3 montraient des inductions similaires pour les 112 gènes sélectionnés (un quatrième conserve cependant une modulation significative).

Sur les personnes restantes, l'expression génique d'une personne reste stable alors que deux ont des rapports d'expression inversés.

Il a été observé que les rapports d'induction restent globalement induits dans le temps (J8-1, J8-2, J12 versus J0) au moins chez les trois personnes parmi quatre prélevées.

### Conclusion :

Globalement, l'analyse de ces deux sélections a mis en évidence des fonctions associées principalement au muscle et à la régulation calcique.

Au final, cette étude a conduit à la classification suivante selon la modulation de l'expression en réponse aux effets du dispositif du générateur d'un champ magnétique alternatif basse fréquence.

Il a été trouvé une réponse forte pour 6 personnes, une réponse significative pour 2 personnes, et pas de réponse pour 3 personnes.

Les champs magnétiques générés par le dispositif de l'invention induisent des contractions musculaires similaires à celles résultant d'un exercice physique soutenu, qui est associé à l'induction d'un ensemble des gènes identifiés dans cette étude.

De plus, la contraction musculaire induite, augmente l'activité de la lipase (HSL) qui après translocation du cytosol vers les gouttelettes lipidiques, favorise l'hydrolyse des triglycérides intramusculaires.

Ainsi, ces observations tendent à démontrer que l'action de l'appareil sur la contraction musculaire est accompagnée d'une redistribution du cholestérol au niveau cellulaire, régulant les fonctions des canaux calciques et le potentiel membranaire.

Ainsi le dispositif de la présente invention possède une action positive sur les lipides accumulés anormalement autour des viscères ou dans les muscles, particulièrement au niveau du tour de taille, ce qui lui confère un rôle positif contre les risques associés au syndrome métabolique.

Le dispositif de la présente invention agit en parallèle sur le déstockage des lipides contenus naturellement dans l'hypoderme, ce qui contribue à utiliser aussi cet appareil pour un remodelage esthétique corporel.

### Exemple 2 : Etude sur le tour de taille des personnes :

En parallèle, il a été évalué la capacité du dispositif de l'invention à induire une diminution du tour de taille, après 12 séances d'une durée de 60 minutes, chez 11 personnes en surpoids ou obèses (obésité sévère ou morbide exclue), non engagés dans un programme amaigrissant.

Cette étude a été réalisée sur les 11 personnes de l'exemple 1.
Au final le tour de taille des volontaires a diminué de plus de 6.0 cm en moyenne après les 12 séances.

Ces résultats confirment donc les résultats de l'étude d'expression génique de l'exemple 1, et les différentes voies métaboliques identifiées comme étant modulées par le dispositif de l'invention.

### Exemple 3 : Etude portant sur l'évaluation des paramètres de la fertilité des patientes stériles en surpoids après une réduction de leur tour de taille par application de la méthode de l'invention

L'étude a été menée au centre de procréation médicalement assistée du service de médecine de la reproduction de l'Hôpital Antoine Béclère devenu en septembre 2010 le centre référant de Fécondation *in vitro* (FIV) pour les obèses en France.

Elle a eu pour objectif d'évaluer l'effet d'une méthode selon l'invention accompagnée d'un régime alimentaire équilibré non amaigrissant, sur les résultats de prise en charge en procréation médicalement assistée par FIV de patientes stériles présentant une obésité abdominale.

La stérilité des patientes est diagnostiquée par le gynécologue traitant après exploration fonctionnelle et réalisation de bilans biologiques conjoint-conjointe (dosage FSH/LH - réserve ovarienne - qualité ovocytaire - spermogramme).

L'étude a porté sur deux groupes de patientes obèses, groupe A comparatif et groupe B.

Chaque patiente du groupe A a suivi le protocole suivant :
- Première visite pour effectuer un bilan sur la réserve ovarienne. Réalisation d'une échographie pelvienne et d'un dosage sanguin. Conseils nutritionnels.
- Deuxième visite 6 semaines après la première pour refaire un contrôle de la présente ovarienne.
- Stimulation ovarienne avec un suivi par échographie et dosages sanguins pour évaluer la réponse ovarienne.
- Ponction ovarienne pour évaluer le nombre d'ovocytes obtenus.
- Fécondation in vitro.

Le groupe A comprenait 109 patientes.

Le groupe B comprenait 17 patientes obèses dont 7 d'obésité de classe 1 (correspondant à un IMC compris entre 30 et 34,9), 5 d'obésité de classe 2 (correspondant à un IMC compris entre 35 à 39,9), et 5 d'obésité de classe 3 (correspondant à un IMC de 40 ou plus). On entend par IMC l'indice de masse corporel. Une patiente est considérée obèse lorsque son IMC est supérieur ou égal à 30.
Le protocole suivi par les patientes du groupe B correspondait à celui des patientes du groupe A ; les patientes du groupe B ont en outre suivi deux séances de traitement selon l'invention, pendant 6 semaines, entre la première et la deuxième visite. Le poids et le tour de taille des patientes de ce groupe ont été mesurés à la première séance, à la sixième séance et à la douzième séance.
Le traitement des patientes a consisté en 12 séances réparties sur 6 semaines avec 2 séances par semaine, chaque séance étant gérée par une infirmière. La durée des séances était de 30 minutes en utilisant la ceinture abdominale décrite précédemment, notamment à la figure 2. L'intensité du courant était de 0,5 A, la fréquence du courant de 50 Hz et la tension égale à 9,9 volts.

Le protocole de fécondation in vitro suivi était classique et était le même pour les deux groupes. Sa description détaillée est disponible auprès de l'établissement hospitalier.

Les résultats préliminaires ont montré une amélioration du taux moyen de grossesse chez les patientes obèses du groupe B ayant suivi la méthode selon l'invention (égal à 41,18%) par rapport au taux moyen de grossesse chez les patientes obèses du groupe de référence A n'ayant pas suivi le traitement (27 %). Sur les 7 grossesses du groupe B, il y a eu deux grossesses spontanées.
Le tour de taille moyen du groupe B a diminué de 5,4 cm à l'issu des 12 séances.

29,41% des patientes du groupe B était en classe 3 contre seulement 2,75% des patientes du groupe A.
La différence du nombre de patientes de classe 3 entre les deux groupes est liée au fait que de nombreuses patientes du groupe A qui était en classe 3 initialement ont suivi un régime alimentaire amaigrissant pour commencer le protocole au moment où elle passait en classe 2, voir directement en classe 1.

Les résultats sont présentés dans le tableau ci-dessous.

| | | Groupe A comparatif | | | Groupe B | | |
|---|---|---|---|---|---|---|---|
| Classification | IMC | Nombre de patientes | Nombre de grossesses | Taux de grossesse | Nombre de patientes | Nombre de grossesses | Taux de grossesse |
| Obésité classe 1 | 30 à 34,9 | 77 | 23 | 29,87% | 7 | 1 | 14,29% |
| Obésité classe 2 | 35 à 39,9 | 29 | 7 | 24,14% | 5 | 4 | 80,00% |
| Obésité classe 3 (obésité morbide) | 40 ou plus | 3 | 0 | 0% | 5 | 2 | 40,00% |
| Nombre total | | 109 | 30 | 27,00% | 17 | 7 | 41,18% |

À l'issue de cette étude, il a donc été démontré une diminution du tour de taille ainsi qu'une amélioration significative du taux de grossesse de patientes obèses, quel que soit la valeur de leur IMC, grâce à la méthode de la présente invention.

## Revendications

1. Dispositif (100 ; 200 ; 300) pour exposer au moins une partie du corps d'une personne à un champ magnétique, **caractérisé en ce qu'**il comprend :
une ceinture isolante (102 ; 202 ; 302) destinée à être appliquée au contact d'une partie du corps de la personne ;
au moins une antenne plate dipolaire (104a, 104b ; 204 ; 304a, 304b) de forme sensiblement rectangulaire émettant un champ magnétique suivant un vecteur (V) perpendiculaire à sa surface, l'antenne étant disposée longitudinalement sur la ceinture dans le sens de la longueur de celle-ci ; et
une source d'alimentation en courant alternatif ou pulsé (106 ; 206 ; 306) de l'antenne,
dans lequel le courant d'alimentation de l'antenne est un courant alternatif présentant une tension comprise entre 5 et 25 V, et une intensité comprise entre 0,3 et 0,8 A, et
dans lequel l'antenne est dimensionnée pour émettre un champ magnétique dont intensité est au maximum de 200 µT;
le ratio entre la longueur et la largeur de l'antenne étant compris entre 5 :1 et 20 :1.

2. Dispositif selon la revendication 1, dans lequel le courant alternatif ou puisé d'alimentation de l'antenne présente une fréquence comprise entre 10 et 100 Hz.

3. Dispositif selon l'une des revendications 1 et 2, dans lequel le courant alternatif ou pulsé d'alimentation de l'antenne présente une fréquence dont la valeur est comprise entre 40 et 60 Hz.

4. Dispositif (100) selon l'une quelconque des revendications 1 à 3, dans lequel la ceinture (102) est une ceinture abdominale ayant deux rangées parallèles d'antennes plates dipolaires, chaque rangée étant composée d'une antenne centrale disposée entre deux antennes latérales, les antennes d'une même rangée étant alimentées en série.

5. Dispositif selon la revendication 4, dans lequel les antennes d'une même rangée sont alimentées en courant alternatif de sorte à ce que l'antenne centrale (104a) émette un champ magnétique suivant un vecteur opposé en direction au vecteur suivant lequel est émis le champ magnétique émis par les antennes latérales (104b).

6. Dispositif (200 ; 300) selon l'une quelconque des revendications 1 à 3, dans lequel la ceinture (202 ; 302) est une ceinture destinée à être portée autour du bras ou du mollet de la personne, ladite ceinture ayant une unique antenne plate dipolaire (204 ; 304b).

7. Dispositif (300) selon l'une quelconque des revendications 1 à 3, dans lequel la ceinture (302) est une ceinture destinée à être portée autour de la cuisse de la personne, ladite ceinture ayant deux rangées parallèles d'antennes plates dipolaires, chaque rangée étant composée de deux antennes latérales (304a) alimentées en série.

8. Dispositif selon l'une quelconque des revendications 1 à 3, se trouvant sous la forme d'un tapis comprenant au moins deux antennes plates dipolaires disposées parallèlement dans le sens de leur longueur.

9. Dispositif selon la revendication 8, dans lequel le tapis comprend au moins quatre antennes plates dipolaires disposées parallèlement dans le sens de leur longueur.

10. Dispositif selon l'une quelconque des revendications 1 à 7, comprenant en outre un boîtier de contrôle de la source d'alimentation en courant alternatif de l'antenne et au moins un capteur de mesure du champ magnétique disposé sur la ceinture et relié audit boîtier de contrôle.

11. Méthode pour simuler une pratique sportive chez une personne, pour relaxer ou pour améliorer la qualité du sommeil, laquelle méthode comprend la mise en oeuvre d'un champ magnétique sur au moins une partie d'au moins un membre et/ou du tronc d'une personne au moyen d'un dispositif tel que décrit à l'une des revendications 1 à 10.

12. Méthode selon la revendication 11, dans laquelle le dispositif est conforme aux revendications 8 ou 9.

13. Méthode selon la revendication 11 ou 13, **caractérisée en ce que** le champ magnétique rayonné par le dispositif possède une fréquence comprise entre 10 et 100 Hertz et une intensité comprise entre 100 et 200 microTesla.

14. Méthode selon la revendication 11 ou 13, **caractérisée en ce qu'**elle comprend un contrôle du dosage du champ magnétique appliqué sur la personne.

15. Méthode selon la revendication 15, **caractérisée en ce qu'**elle comprend la disposition d'un capteur d'ondes magnétiques à proximité immédiate d'une antenne rayonnante, ledit capteur d'ondes magnétiques étant en communication avec un boîtier de contrôle du champ magnétique, ledit boîtier de contrôle communiquant avec une ou plusieurs antennes rayonnantes, et la mesure par le boîtier de contrôle de la perméabilité magnétique de la personne afin d'adapter le champ magnétique émis par le ou les antennes rayonnantes en fonction d'un profil d'individu.

16. Méthode selon la revendication 11 ou 13, **caractérisée en ce qu'**elle comprend une mesure de la réponse des tissus de la personne à une mise en contact avec un champ magnétique, ladite mesure comprenant la variation des fréquences d'un champ électromagnétique, de préférence entre 10Hz et 10kHz, au regard d'une ou plusieurs parties d'un ou plusieurs membres et/ou d'une ou plusieurs parties du tronc de la personne, et la mesure du champ électromagnétique pour estimer la réponse des tissus de la personne.

17. Méthode selon la revendication 17, **caractérisée en ce qu'**elle comprend l'estimation de la masse graisseuse et/ou de la masse musculaire de la personne afin notamment d'adapter l'intensité et/ou la durée du champ magnétique appliqué à la personne.

18. Méthode selon l'une quelconque des revendications 17 ou 18, **caractérisée en ce que** la mesure est effectuée par l'intermédiaire d'un capteur hall ou magnétorésistif, notamment pour déterminer le seuil de puissance maximum et/ou optimum du champ magnétique à appliquer à la personne.

## Patentansprüche

1. Vorrichtung (100; 200; 300), um wenigstens einen Teil des Körpers einer Person einem Magnetfeld auszusetzen, **dadurch gekennzeichnet, dass** sie umfasst:
einen Isoliergürtel (102; 202; 302), der dazu bestimmt ist, in Kontakt mit einem Teil des Körpers der Person angelegt zu werden;
wenigstens eine flache Dipolantenne (104a, 104b; 204; 304a, 304b) mit im Wesentlichen rechteckiger Form, die ein Magnetfeld entlang eines Vektors (V) senkrecht zu ihrer Oberfläche aussendet, wobei die Antenne auf dem Gürtel in der Richtung seiner Länge längs angeordnet ist, und
eine Quelle (106; 206; 306) zur Versorgung der Antenne mit Wechsel- oder Impulsstrom,
wobei der Speisestrom der Antenne ein Wechselstrom ist, der eine Spannung im Bereich zwischen 5 und 25 V und eine Stärke im Bereich zwischen 0,3 und 0,8 A aufweist, und
wobei die Antenne dimensioniert ist, um ein Magnetfeld auszusenden, dessen Stärke maximal 200 µT beträgt;
wobei das Verhältnis zwischen der Länge und der Breite der Antenne im Bereich zwischen 5 : 1 und 20 : 1 liegt.

2. Vorrichtung nach Anspruch 1, wobei der Wechsel- oder Impulsspeisestrom der Antenne eine Frequenz im Bereich zwischen 10 und 100 Hz aufweist.

3. Vorrichtung nach einem der Ansprüche 1 und 2, wobei der Wechsel- oder Impulsspeisestrom der Antenne eine Frequenz aufweist, deren Wert im Bereich zwischen 40 und 60 Hz liegt.

4. Vorrichtung (100) nach einem der Ansprüche 1 bis 3, wobei der Gürtel (102) ein Bauchgürtel mit zwei parallelen Reihen von flachen Dipolantennen ist, wobei jede Reihe aus einer mittleren Antenne, die zwischen zwei Seitenantennen angeordnet ist, besteht, wobei die Antennen einer gleichen Reihe in Reihe gespeist werden.

5. Vorrichtung nach Anspruch 4, wobei die Antennen einer gleichen Reihe mit Wechselstrom gespeist werden, so dass die mittlere Antenne (104a) ein Magnetfeld entlang eines Vektors aussendet, der richtungstechnisch zu dem Vektor, entlang dessen das durch die Seitenantennen (104b) ausgesandte Magnetfeld ausgesendet wird, entgegengesetzt ist.

6. Vorrichtung (200; 300) nach einem der Ansprüche 1 bis 3, wobei der Gürtel (202; 302) ein Gurt ist, der dazu bestimmt ist, um den Arm oder die Wade der Person getragen zu werden, wobei der Gurt eine einzige flache Dipolantenne (204; 304b) aufweist.

7. Vorrichtung (300) nach einem der Ansprüche 1 bis 3, wobei der Gürtel (302) ein Gurt ist, der dazu bestimmt ist, um den Oberschenkel der Person getragen zu werden, wobei der Gurt zwei parallele Reihen von flachen Dipolantennen aufweist, wobei jede Reihe aus zwei in Reihe gespeisten Seitenantennen (304a) besteht.

8. Vorrichtung nach einem der Ansprüche 1 bis 3, die in Form einer Matte vorliegt, die wenigstens zwei flache Dipolantennen, welche in der Richtung ihrer Länge parallel angeordnet sind, umfasst.

9. Vorrichtung nach Anspruch 8, wobei die Matte wenigstens vier flache Dipolantennen, die in der Richtung ihrer Länge parallel angeordnet sind, umfasst.

10. Vorrichtung nach einem der Ansprüche 1 bis 7, ferner umfassend ein Gehäuse zur Steuerung der Wechselstromversorgungsquelle der Antenne sowie wenigstens einen Sensor zur Messung des Magnetfeldes, der auf dem Gürtel angeordnet und mit dem Steuergehäuse verbunden ist.

11. Verfahren zum Simulieren einer sportlichen Tätigkeit bei einer Person, um zu entspannen oder um die Schlafqualität zu verbessern, wobei das Verfahren den Einsatz eines Magnetfeldes an wenigstens einem Teil wenigstens einer Gliedmaße und/oder des Rumpfes einer Person mittels einer Vorrichtung, wie sie in einem der Ansprüche 1 bis 10 beschrieben ist, umfasst.

12. Verfahren nach Anspruch 11, wobei die Vorrichtung gemäß den Ansprüchen 8 oder 9 ist.

13. Verfahren nach Anspruch 11 oder 13, **dadurch gekennzeichnet, dass** das durch die Vorrichtung ausgestrahlte Magnetfeld eine Frequenz im Bereich zwischen 10 und 100 Hertz und eine Stärke im Bereich zwischen 100 und 200 Mikrotesla besitzt.

14. Verfahren nach Anspruch 11 oder 13, **dadurch gekennzeichnet, dass** es ein Steuern der Dosierung des auf die Person angewandten Magnetfeldes umfasst.

15. Verfahren nach Anspruch 15, **dadurch gekennzeichnet, dass** es die Anordnung eines Magnetwellen-Sensors in unmittelbarer Nähe einer strahlenden Antenne, wobei der Magnetwellen-Sensor mit einem Gehäuse zur Steuerung des Magnetfeldes in Verbindung ist, wobei das Steuergehäuse mit einer oder mehreren strahlenden Antennen verbunden ist, sowie die Messung der magnetischen Permeabilität der Person durch das Steuergehäuse, um das durch die strahlende(n) Antenne oder Antennen ausgesandte Magnetfeld in Abhängigkeit eines Personenprofils anzupassen, umfasst.

16. Verfahren nach Anspruch 11 oder 13, **dadurch gekennzeichnet, dass** es eine Messung der Reaktion der Gewebe der Person auf ein Inkontaktbringen mit einem Magnetfeld, wobei die Messung die Änderung der Frequenzen eines elektromagnetischen Feldes, vorzugsweise zwischen 10 Hz und 10 kHz, im Hinblick auf einen oder mehrere Teile einer oder mehrerer Gliedmaßen und/oder einen oder mehrere Teile des Rumpfes der Person umfasst, sowie die Messung des elektromagnetischen Feldes, um die Reaktion der Gewebe der Person zu bewerten, umfasst.

17. Verfahren nach Anspruch 17, **dadurch gekennzeichnet, dass** es die Schätzung der Fettmasse und/oder der Muskelmasse der Person umfasst, um insbesondere die Stärke und/oder die Dauer des auf die Person angewandten Magnetfeldes anzupassen.

18. Verfahren nach einem der Ansprüche 17 oder 18, **dadurch gekennzeichnet, dass** die Messung mittels eines Hall- oder magnetoresistiven Sensors durchgeführt wird, insbesondere um die Schwelle maximaler und/oder optimaler Leistung des auf die Person anzuwendenden Magnetfeldes zu bestimmen.

## Claims

1. A device (100; 200; 300) for exposing at least a part of the body of a person to a magnetic field, **characterized in that** it comprises:
an insulating belt (102; 202; 302) intended to be applied in contact with a part of the body of the person;
at least one substantially rectangular flat dipolar antenna (104a, 104b; 204; 304a, 304b) emitting a magnetic field on a vector (V) perpendicular to its surface, the antenna being positioned longitudinally on the belt in the lengthwise direction thereof; and
an alternating or pulsed current power supply source (106; 206; 306) for the antenna,
in which the power supply current for the antenna is an alternating current having a voltage of between 5 and 25 V, and an intensity of between 0.3 and 0.8 A,
in which the antenna is dimensioned to emit a magnetic field with a maximum intensity of 200 µT, and
the ratio between the length and the width of the antenna is 5:1 and 20:1.

2. The device as claimed in claim 1, in which the alternating or pulsed power supply current for the antenna has a frequency of between 10 and 100 Hz.

3. The device as claimed in one of claims 1 and 2, in which the alternating or pulsed power supply current for the antenna has a frequency with a value of between 40 and 60 Hz.

4. The device (100) as claimed in any one of claims 1 to 3, in which the belt (102) is an abdominal belt having two parallel rows of flat dipolar antennas, each row consisting of a central antenna positioned between two lateral antennas, the antennas of one and the same row being supplied in series.

5. The device as claimed in claim 4, in which the antennas of one and the same row are supplied with alternating current in such a way that the central antenna (104a) emits a magnetic field on a vector opposite in direction to the vector on which the magnetic field emitted by the lateral antennas (104b) is emitted.

6. The device (200; 300) as claimed in any one of claims 1 to 3, in which the belt (202; 302) is a belt intended to be worn around the arm or the calf of the person, said belt having a single flat dipolar antenna (204; 304b).

7. The device (300) as claimed in any one of claims 1 to 3, in which the belt (302) is a belt intended to be worn around the thigh of the person, said belt having two parallel rows of flat dipolar antennas, each row consisting of two lateral antennas (304a) supplied in series.

8. The device as claimed in any one of claims 1 to 3, having the form of a mat comprising at least two flat dipolar antennas positioned parallel in their lengthwise direction.

9. The device as claimed in claim 8, in which the mat comprises at least four flat dipolar antennas positioned parallel in their lengthwise direction.

10. The device as claimed in any one of claims 1 to 7, also comprising a module for controlling the alternating current power supply source for the antenna and at least one magnetic field measurement sensor positioned on the belt and linked to said control module.

11. A method for simulating a sporting activity in a person, for relaxing or enhancing sleep quality, said method comprising the application of a magnetic field to at least a part of at least one member and/or the trunk of a person by means of a device as described in one of claims 1 to 10.

12. The method as claimed in claim 11, in which the device conforms to claims 8 or 9.

13. The method as claimed in claim 11 or 13, **characterized in that** the magnetic field radiated by the device has a frequency of between 10 and 100 hertz and an intensity of between 100 and 200 microTesla.

14. The method as claimed in claim 11 or 13, **characterized in that** it comprises control of the dosage of the magnetic field applied to the person.

15. The method as claimed in claim 15, **characterized in that** it comprises the positioning of a magnetic wave sensor in immediate proximity to a radiating antenna, said magnetic wave sensor being in communication with a magnetic field control module, said control module communicating with one or more radiating antennas, and the measurement by the control module of the magnetic permeability of the person in order to adapt the magnetic field emitted by the radiating antenna or antennas according to an individual profile.

16. The method as claimed in claim 11 or 13, **characterized in that** it comprises a measurement of the response of the tissues of the person to contact with a magnetic field, said measurement comprising the variation of the frequencies of an electromagnetic field, preferably between 10 Hz and 10 kHz, with respect to one or more parts of one or more members and/or one or more parts of the trunk of the person, and the measurement of the electromagnetic field to estimate the response of the tissues of the person.

17. The method as claimed in claim 17, **characterized in that** it comprises the estimation of the fatty mass and/or of the muscular mass of the person in order in particular to adapt the intensity and/or the duration of the magnetic field applied to the person.

18. The method as claimed in either of claims 17 or 18, **characterized in that** the measurement is performed via a Hall or magnetoresistive sensor, in particular to determine the maximum and/or optimum power threshold of the magnetic field to be applied to the person.
